# EUROPEAN PATENT APPLICATION

(11) **EP 2 698 101 A1**
(43) Date of publication of application: **19.02.2014**
(21) Application number: 13180325.6
(22) Date of filing: 14.08.2013
(51) Int. Cl.: A61B 5/00, A61B 5/055

(54) **Mri imaging system for generating a rendered image**

(30) Priority: 15.08.2012 IL 22149012
(71) Applicant: Aspect Imaging Ltd., 60850 Shoham (IL)
(72) Inventor: Batt, Aryeh, 90440 Har Hevron (IL); Rapoport, Uri, 73115 Moshav Ben Shemen (IL)
(74) Representative: Lecomte & Partners

(57) **Abstract**

The present application is directed to an MRI imaging system having an MRI device for imaging at least a portion of an animal. The system comprises a photon transmitter, introducible within the body of an animal; at least one imaging photon detector located either within or outside said animal, for detecting fluorescence excited within said animal by said transmitted photons; and an image processor adapted to superimpose said MRI image and said at least one photon detector image, generating a rendered MRI image of said at least a portion of said animal. Further, the present application relates to a method using the system for MRI imaging.

## Description

### FIELD OF THE INVENTION

The present invention generally pertains to a system and method for an MRI-based system for generating a rendered image of an interior portion of an animal by superimposing an MRI image and a fluorescence image, where the fluorescence is excited by light emitted by a photon emitter located within an animal.

### BACKGROUND OF THE INVENTION

*In vivo* fluorescence imaging uses a sensitive camera to detect fluorescence emission from fluorophores in whole-body living small animals. To overcome the photon attenuation in living tissue, fluorophores with long emission at the near-infrared (NIR) region are generally preferred, including widely used small indocarbocyanine dyes.

Molecules that absorb in the near infrared (NIR) region, 700-1,000 nm, can be efficiently used to visualize and investigate *in vivo* molecular targets because most tissues generate little NIR fluorescence. The most common organic NIR fluorophores are polymethines. Among them, pentamethine and heptamethine cyanines comprising benzoxazole, benzothaizole, indolyl, 2-quinoline or 4-quinoline have been found to be the most useful.

Fluorescence images enable determination of cells types, cell activity and protein activity; See review by Lowry et al., Molecular Fluorescence, Phosphorescence, and Chemiluminescence Spectrometry Analytical Chemistry, Vol. 80, No. 12, June 15, 4551-4574, 2008, which is incorporated herein as a reference.

MRI or NMR provides images of the locations of particular atomic species in a volume of interest, especially protons. MRI provides good contrast between the different soft tissues of the body, which makes it especially useful in imaging the brain, muscles, the heart, and cancers compared with other medical imaging techniques such as computed tomography (CT) or X-rays. MRI contrast agents may be injected intravenously to enhance the appearance of blood vessels, tumors or inflammation. Contrast agents may also be directly injected into a joint in the case of arthrograms, MRI images of joints.

Patent application US 2010/0113902 discloses an efficient, effective, MRI compatible small bore MRI noninvasive photoplefhysmographic sensor for animals such as small rodents, namely rats and mice. The photoplethysmographic sensor for animals comprising: a nonmagnetic sensor coupling attachable to an animal; fiber optic cable coupled to the sensor coupling and configured to deliver a signal to and receive a signal from the animal tissue adjacent the sensor coupling; an opto-electical converter coupled to the fiber optic cable, the converter including a receiver coupled the fiber optic cable portion configured to receive a signal from the animal tissue and including an emitter coupled to the fiber optic portion configured to deliver a signal to the animal tissue; an electronic coupling extending from the opto-electric converter and configured to be coupled to the emitter and the receiver, wherein the electronic coupling is configured to extend outside of the MRI chamber; and a processor coupled to the electronic coupling. However, the energy inducing the fluorescence within the animal is supplied from a source attached to the outside of the animal.

Patent application US 2005/0028482 discloses systems and methods for multi-modal imaging with light and a second form of imaging. Light imaging involves the capture of low intensity light from a light-emitting object. A camera obtains a two-dimensional spatial distribution of the light emitted from the surface of the subject. Software operated by a computer in communication with the camera may then convert two-dimensional spatial distribution data from one or more images into a three-dimensional spatial representation. The second imaging mode may include any imaging technique that compliments light imaging. Examples include magnetic resonance imaging (MRI) and computer topography (CT). An object handling system moves the object to be imaged between the light imaging system and the second imaging system, and is configured to interface with each system. However, the energy inducing the fluorescence within the animal is supplied from a source external to the animal.

It is therefore a long felt need to provide a multimodal system combining MRI and fluorescence for preclinical and clinical investigations of tumors where the source of energy used to induce fluorescence is not located outside the body.

### SUMMARY OF THE INVENTION

It is an object of the present invention to disclose a system and method for an MRI-based system for generating a rendered image of an interior portion of an animal by superimposing an MRI image and a fluorescence image, where the fluorescence is excited by light emitted by a photon emitter located within the animal.

It is an object of the present invention to disclose an MRI imaging system, having an MRI device for imaging at least a portion of an animal, the system characterized by a photon transmitter, introducible within the body of an animal; at least one imaging photon detector located either within or outside the animal, for detecting fluorescence excited within the animal by the transmitted photons; and an image processor adapted to superimpose the MRI image and the at least one photon detector image, generating a rendered MRI image of the at least a portion of the animal.

It is an object of the present invention to disclose the MRI system, wherein the transmitter of photons is selected from the group consisting of an optical fiber, a cannula, a light pipe, a light tube, and any combination thereof.

It is an object of the present invention to disclose the MRI system, wherein the optical fiber is selected from the group consisting of silica glass fiber, fluorozirconate glass fiber, fluoroaluminate glass fiber, chalcogenide glass fiber, sapphire fiber, and polymer optical fiber.

It is an object of the present invention to disclose the MRI system, wherein the transmitter of photons enters the body through an orifice selected from the group consisting of a cannula inserted in the animal, a trocar inserted in the animal, a laparoscopy system inserted in the animal, the nose, the mouth, the anus, the vagina, the urethra, the ear, and any combination thereof.

It is an object of the present invention to disclose the MRI system, wherein the photons are in at least one range selected from the group consisting of X-rays, far ultraviolet, near ultraviolet, visible light, near infrared and far infrared.

It is an object of the present invention to disclose the MRI system, wherein the at least one photon detector is selected from the group consisting of a CCD array, a camera, a photoconductive detector array, a photovoltaic detector array, a quantum dot array, a superconducting single-photon detector array, a photovoltaic cell array, a phototube array, and any combination thereof.

It is an object of the present invention to disclose the MRI system, wherein the image processor is adapted to render the superimposed image by a Boolean method of correlating or combining at least a portion of the MRI image and at least a portion of the photon detector image.

It is an object of the present invention to disclose the MRI system, wherein the Boolean method uses Boolean operators selected from the group consisting of OR, AND, NOT, EXCLUSIVE OR and any combination thereof.

It is an object of the present invention to disclose the MRI system, wherein magnets in the MRI imaging system are selected from the group consisting of permanent magnets, superconducting magnets, and any combination thereof.

It is an object of the present invention to disclose a method for MRI imaging at least a portion of an animal, comprising steps of providing an MRI imaging system, having an MRI device for imaging at least a portion of an animal; the system characterized by a photon transmitter, introducible within the body of an animal; at least one imaging photon detector located either within or outside the animal, for detecting fluorescence excited within the animal by the transmitted photons; and an image processor adapted to superimpose the MRI image and the photon detector image, generating a rendered MRI image of the at least a portion of the animal; introducing the photon transmitter within the body of an animal; MRI-imaging the at least a portion of the animal; creating a photon detector image of the at least a portion of the animal; and superimposing the MRI image and the photon detector image, generating a rendered MRI image of the at least a portion of the animal.

It is an object of the present invention to disclose the method, additionally comprising a step of selecting the transmitter of photons from the group consisting of an optical fiber, a cannula, a light pipe, a light tube, and any combination thereof.

It is an object of the present invention to disclose the method, additionally comprising a step of selecting the optical fiber from the group consisting of silica glass fiber, fluorozirconate glass fiber, fluoroaluminate glass fiber, chalcogenide glass fiber, sapphire fiber, and polymer optical fiber

It is an object of the present invention to disclose the method, additionally comprising a step of emplacing the fiber within the body through an orifice selected from the group consisting of a cannula inserted in the animal, a trocar inserted in the animal, a laparoscopy system inserted in the animal, the nose, the mouth, the anus, the vagina, the urethra, the ear, and any combination thereof.

It is an object of the present invention to disclose the method, additionally comprising a step of selecting the range of the photons from at least one of the group consisting of X-rays, far ultraviolet, near ultraviolet, visible light, near infrared and far infrared.

It is an object of the present invention to disclose the method, additionally comprising a step of selecting the at least one photon detector from the group consisting of a CCD array, a camera, a photoconductive detector array, a photovoltaic detector array, a quantum dot array, a superconducting single-photon detector array, a photovoltaic cell array, a phototube array, and any combination thereof.

It is an object of the present invention to disclose the method, additionally comprising a step of rendering the superimposed image by a Boolean method of correlating or combining at least a portion of the MRI image and at least a portion of the photon detector image.

It is an object of the present invention to disclose the method, additionally comprising a step of selecting Boolean operators in the Boolean method from the group consisting of OR, AND, NOT, EXCLUSIVE OR and any combination thereof.

It is an object of the present invention to disclose the method, additionally comprising a step of selecting magnets in the MRI imaging system from the group consisting of permanent magnets, superconducting magnets, and any combination thereof.

### BRIEF DESCRIPTION OF THE FIGURES

In order to better understand the invention and its implementation in practice, a plurality of embodiments will now be described, by way of non-limiting example only, with reference to the accompanying drawings, wherein
Fig. 1 schematically illustrates an embodiment of the system of the present invention; and
Fig. 2 depicts a block diagram of a method of using the system of the present invention.

### DETAILED DESCRIPTION OF THE PREFERRED EMBODIEMNTS

The following description is provided, alongside all chapters of the present invention, so as to enable any person skilled in the art to make use of the invention and sets forth the best modes contemplated by the inventor of carrying out this invention. Various modifications, however, will remain apparent to those skilled in the art, since the generic principles of the present invention have been defined specifically to provide an MRI-based system and method for generating a rendered image of at least a portion of a living subject.

*In vivo* fluorescence imaging uses a sensitive camera to detect fluorescence emission from fluorophores in whole-body living small animals. To overcome the photon attenuation in living tissue, fluorophores with long emission at the near-infrared (NIR) region, 700-1000 nm, are generally preferred because they can be efficiently used to visualize and investigate *in vivo* molecular targets, since most tissues generate little NIR fluorescence.

One widely used class of fluorophores is small indocarbocyanine dyes. In addition, fluorescent organic, inorganic and biological nanoparticles are used. Another class of probes for *in vivo* fluorescence imaging is semiconductor nanocrystals or quantum dots. QDs that emit at several different wavelengths can be excited with a single wavelength, and thus are suitable for multiplex detection of multiple targets in a single experiment.

Magnetic resonance imaging (MRI) uses a strong static magnetic field to align nuclear magnetic moment with the field. A varying field, usually with frequencies in the range of 50 - 200 MHz, is used to systematically alter the alignment of this magnetization. This causes the nuclei to produce a rotating magnetic field detectable by the scanner, and this information is recorded to construct an image of the scanned area of the body. Magnetic field gradients cause nuclei at different locations to rotate at different speeds. By using gradients in different directions, 2D images or 3D volumes can be obtained in any arbitrary orientation. MRI provides good contrast between the different soft tissues of the body, which makes it especially useful in imaging the brain, muscles, the heart, and cancers compared with other medical imaging techniques such as computed tomography (CT) or X-rays. Unlike CT scans or traditional X-rays, MRI does not use ionizing radiation.

Fluorescence images of the tumors enable determination of cells types (senescent, aggressive, etc.) and protein activity, while NMR/MRI enables determination of the location of, especially, protons. Differences between bound and free water, for example, allow determination of edema.

MRI provides good contrast between the different soft tissues of the body, which makes it especially useful in imaging the brain, muscles, the heart, and cancers compared with other medical imaging techniques such as computed tomography (CT) or X-rays. MRI contrast agents may be injected intravenously to enhance the appearance of blood vessels, tumors or inflammation. Contrast agents may also be directly injected into a joint in the case of arthrograms, MRI images of joints.

The system of the present invention provides a system of simultaneously acquiring MRI images and fluorescence images in living subjects of organs, tumors, blood vessels, nerves, or any other objects in the living subject that can be made to fluoresce.

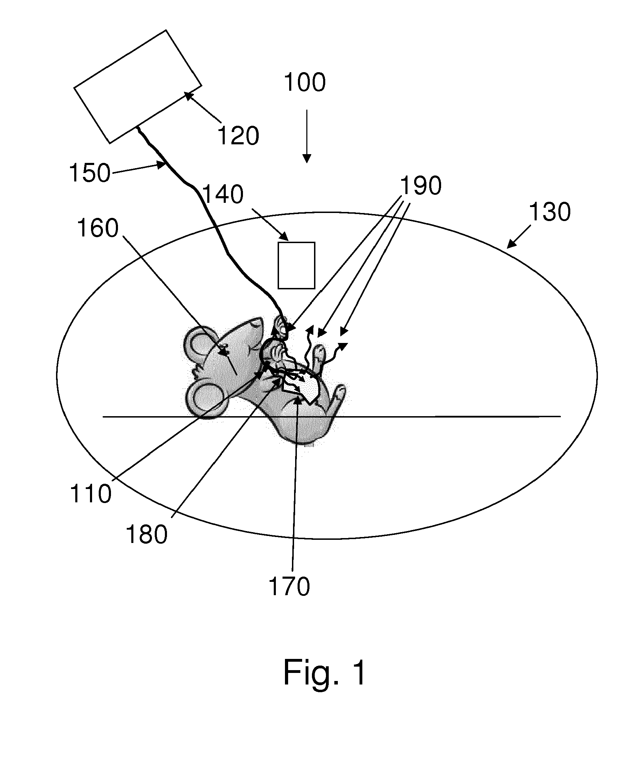

In reference to **Fig. 1**, which shows one embodiment (100) of the system, the subject (110), or the portion of the subject containing the volume of interest, is placed within an MRI imaging device (130). An optical fiber (150) connectable to an appropriate light source (120) is passed into the subject through incision 160 to a position in proximity with the volume of interest (170), where the provided light (180) causes fluorescence (190) of fluorescent material in the volume of interest. This fluorescent light is detected with sensors (140) outside the body of the subject.

The optical fiber (150) can be introduced into the body through a cannula or a trocar, either an independent cannula or trocar or one forming part of a laparoscopy system, or it can lie within an incision without trocar or cannula. It can also be introduced via a body orifice, such as the nose, mouth, anus, vagina, or urethra, or via a body orifice, as given above, and through a body tissue, either via an incision or through a cannula. One example of the last would be positioning the optical fiber within the skull by passing it via the nasal passages to the ethmoid bone and through the ethmoid bone to the interior of the skull.

In the system of the present invention, the MRI images, which take several seconds to several minutes to acquire, provide structural information about body parts, such as organs, blood vessels, or tumors, in the volume of interest, while the fluorescence images, which may take a second or less to acquire, provide functional information about the body part or parts. For non-limiting example, MRI images show the shape and size of a tumor, while fluorescence image shows the locations of apoptopic cells and aggressively dividing cells within it.

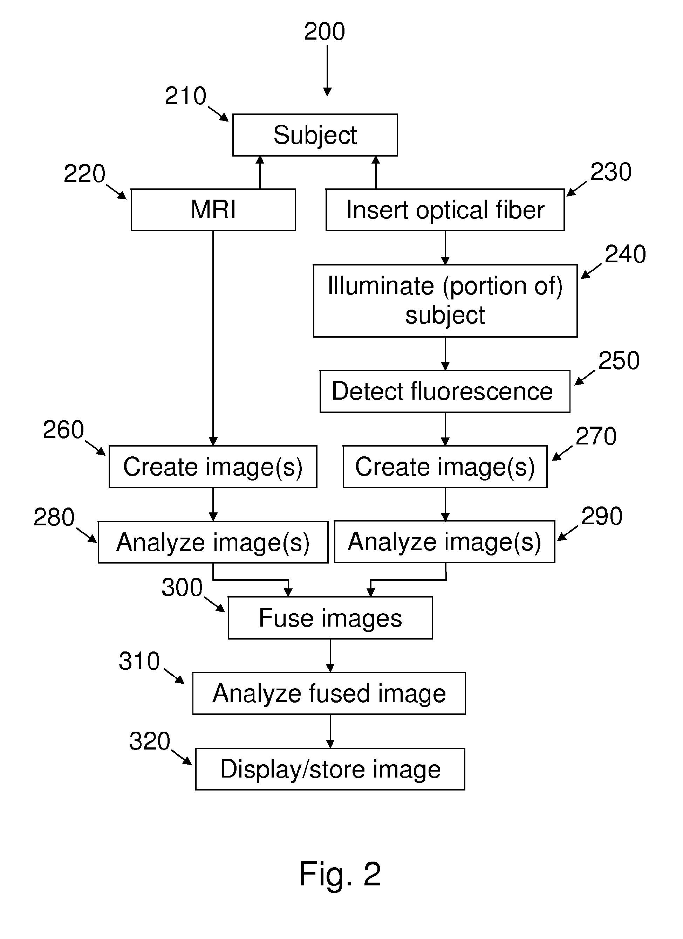

In reference to **Fig. 2**, a block diagram (200) of an embodiment of a method of using the system is shown. At least a volume of interest within the subject is placed within the system. The volume of interest can be the entire subject or a portion thereof, such as an organ or a tumor within the subject, a set of blood vessels or a set of nerves. An optical fiber is emplaced within the subject (230) in such a position that light from the fiber will activate fluorescent material within the volume of interest inside the subject. The fluorescent material can be material introduced into the subject by any of the means well known in the art, or it can be fluorescent material produced by the subject. The volume of interest is illuminated (240) via the optical fiber and the resulting fluorescence is detected by a detector outside the body of the subject (250). An image or images are created (270) of the volume of interest, using the detected fluorescence, and the image or images are analyzed (290).

An MRI scan or scans of the volume of interest in the subject is made (220). An image or images is created (260) of the volume of interest from the MRI scan, and the image or images are analyzed (280).

The MRI and fluorescence images are then fused (300), using techniques well known in the art, and the combined image is analyzed (310) and displayed or stored for later use (320).

Fusing techniques include rendering the images using Boolean methods of correlating and combining the images. Combining binary images using Boolean logic makes it possible to select structures or objects based on multiple criteria, such as, but not limited to, masking and threshholding. The Boolean operators commonly used are OR, AND, NOT, EXCLUSIVE OR and combinations thereof.

Optical fibers are most commonly silica glass, but can also be made from fluorozirconate glass, fluoroaluminate glass, chalcogenide glass, sapphire, and polymers. The most common polymer optical fibers (POF) are (1) polymethylmethacrilate (PMMA) core with fluorinated polymer cladding, although other POF include: PMMA or Polystyrene core with silicone resin cladding, perfluorinated polymer (mainly polyperfluorobutenylvinylether) POFs, and microstructured polymer optical fibers (mPOF), which are a type of photonic crystal fiber.

## Claims

1. An MRI imaging system, having an MRI device for imaging at least a portion of an animal, said system **characterized by**
a. a photon transmitter, introducible within the body of an animal;
b. at least one imaging photon detector located either within or outside said animal, for detecting fluorescence excited within said animal by said transmitted photons; and
c. an image processor adapted to superimpose said MRI image and said at least one photon detector image, generating a rendered MRI image of said at least a portion of said animal.

2. The MRI imaging system of claim 1, wherein the transmitter of photons is selected from the group consisting of an optical fiber, a cannula, a light pipe, a light tube, and any combination thereof.

3. The MRI imaging system of claim 2, wherein said optical fiber is selected from the group consisting of silica glass fiber, fluorozirconate glass fiber, fluoroaluminate glass fiber, chalcogenide glass fiber, sapphire fiber, and polymer optical fiber.

4. The MRI imaging system of claim 1, wherein said transmitter of photons enters the body through an orifice selected from the group consisting of a cannula inserted in the animal, a trocar inserted in the animal, a laparoscopy system inserted in the animal, the nose, the mouth, the anus, the vagina, the urethra, the ear, and any combination thereof.

5. The MRI imaging system of claim 1, wherein said photons are in at least one range selected from the group consisting of X-rays, far ultraviolet, near ultraviolet, visible light, near infrared and far infrared.

6. The MRI imaging system of claim 1, wherein said at least one photon detector is selected from the group consisting of a CCD array, a camera, a photoconductive detector array, a photovoltaic detector array, a quantum dot array, a superconducting single-photon detector array, a photovoltaic cell array, a phototube array, and any combination thereof.

7. The MRI imaging system of claim 1, wherein said image processor is adapted to render said superimposed image by a Boolean method of correlating or combining at least a portion of said MRI image and at least a portion of said photon detector image.

8. The MRI imaging system of claim 5, wherein said Boolean method uses Boolean operators selected from the group consisting of OR, AND, NOT, EXCLUSIVE OR and any combination thereof.

9. The MRI imaging system of claim 8, wherein magnets in said MRI imaging system are selected from the group consisting of permanent magnets, superconducting magnets, and any combination thereof.

10. A method for MRI imaging at least a portion of an animal, comprising steps of:
a. providing an MRI imaging system, having an MRI device for imaging at least a portion of an animal; said system **characterized by**
i. photon transmitter, introducible within the body of an animal;
ii. at least one imaging photon detector located either within or outside said animal, for detecting fluorescence excited within said animal by said transmitted photons; and
iii. an image processor adapted to superimpose said MRI image and said photon detector image, generating a rendered MRI image of said at least a portion of said animal;
b. introducing said photon transmitter within the body of an animal;
c. MRI-imaging said at least a portion of said animal;
d. creating a photon detector image of said at least a portion of said animal; and
e. superimposing said MRI image and said photon detector image, generating a rendered MRI image of said at least a portion of said animal.

11. The method of claim 10, additionally comprising a step of selecting the transmitter of photons from the group consisting of an optical fiber, a cannula, a light pipe, a light tube, and any combination thereof.

12. The method of claim 11, additionally comprising a step of selecting the optical fiber from the group consisting of silica glass fiber, fluorozirconate glass fiber, fluoroaluminate glass fiber, chalcogenide glass fiber, sapphire fiber, and polymer optical fiber

13. The method of claim 10, additionally comprising at least one step selected from a group consisting of (a) selecting said at least one photon detector from the group consisting of a CCD array, a camera, a photoconductive detector array, a photovoltaic detector array, a quantum dot array, a superconducting single-photon detector array, a photovoltaic cell array, a phototube array, and any combination thereof; (b) selecting magnets in said MRI imaging system from the group consisting of permanent magnets, superconducting magnets, and any combination thereof; (c) selecting the range of said photons from at least one of the group consisting of X-rays, far ultraviolet, near ultraviolet, visible light, near infrared and far infrared; (d) emplacing said fiber within said body through an orifice selected from the group consisting of a cannula inserted in the animal, a trocar inserted in the animal, a laparoscopy system inserted in the animal, the nose, the mouth, the anus, the vagina, the urethra, the ear, and any combination thereof; and any combination thereof.

14. The method of claim 10, additionally comprising a step of rendering said superimposed image by a Boolean method of correlating or combining at least a portion of said MRI image and at least a portion of said photon detector image.

15. The method of claim 14, additionally comprising a step of selecting Boolean operators in said Boolean method from the group consisting of OR, AND, NOT, EXCLUSIVE OR and any combination thereof.
